# EUROPEAN PATENT APPLICATION

(11) **EP 4 001 396 A1**
(43) Date of publication of application: **25.05.2022**
(21) Application number: 20208112.1
(22) Date of filing: 17.11.2020
(51) Int. Cl.: C12M 3/00, C12M 1/00, C12M 1/42

(54) **DEVICE FOR TREATING CELLS IN A BYPASS**

(71) Applicant: Bühler AG, 9240 Uzwil (CH)
(72) Inventor: Buchmann, Leandro, 8408 Winterthur (CH); Senn, Michael, 9000 St. Gallen (CH)
(74) Representative: Hepp Wenger Ryffel AG

(57) **Abstract**

The present invention is related to a device (1), comprising a unit (2) for taking up a fluid, a unit (3) for emitting electric pulses, wherein said unit (2) for taking up a fluid and said unit (3) for emitting electric pulses are connected with each other via lines (4a, 4a', 4b, 4b') so as to form a circuit, wherein there are provided connections (5a, 5b) for connecting the lines (4a, 4b) arranged at said unit (2) for taking up a fluid with the lines (4a', 4b') arranged at said unit (3) for emitting electric pulses in a releasable, hygienic and leakage-free manner. The present invention is related to an apparatus (9) comprising said device (1), and to a method for treating cells performed with said device (1) or said apparatus (9).

## Description

The present invention is related to a device for treating cells for medical, environmental, food applications, and bio-based industries (including yeast, lactobacilli, algae, and cell tissue production systems, in particular including targeted inactivation, the extraction of bioactive compounds, and the stimulation of cell growth and/or cellular compounds, wherein said treatment is conducted in a bypass, and to a method for treating cells in such a device.

It is known that prokaryotic and eukaryotic cells are influenced by the action of electric fields. Stimulation of cell growth, as well as cell death, inactivation of microorganisms, or specific extraction of cell constituents can occur, depending on the applied electric field strength (e.g. Buchmann L and Mathys A (2019), Perspective on Pulsed Electric Field Treatment in the Bio-based Industry, Front. Bioeng. Biotechnol. 7:265, doi: 10.3389/fbioe.2019.00265).

EP-2 308 969 B1 describes a PEF (pulsed electric field) method where a cell material suspended in an electrically conductive liquid, the cell material being positioned between two electrodes, by exposure of 1 to 100 electric field strength pulses, such that a voltage increase takes place between the two electrodes of 10% to 90% of a target voltage of the electric field strength pulses within a period of 0.1 to 100 ns, the electric field strength pulses have a pulse duration of 5 ns to 5000 ns, and the electric field strength pulses, upon reaching the target voltage, have an electric field strength of 0.5 kV/cm to 50 kV/cm, showed an accelerated cell proliferation and/or increased cell constituents.

In the device for treating cell material used in EP 2 308 969 B1, an electroporation cuvette having the suspension of cell material described above was continuously pumped through an arrangement of two electrodes and exposed to an electric field in an electrically conductive liquid (paragraph [0020] of EP-2 308 969 B1).

In the prior art, it has been proposed to provide the material to be treated in a vessel (also called bioreactor), and for treatment transfer the material in a line that is connected with an outlet of said vessel. The other end of said line is connected with an inlet into said vessel, so that the vessel and the line form a fluid circuit. Said line is also called bypass.

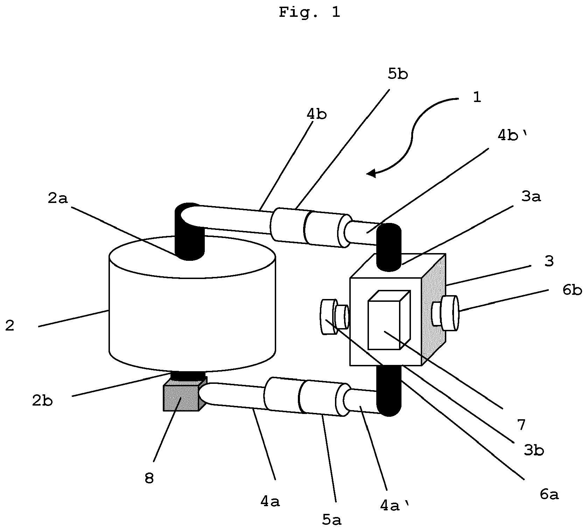

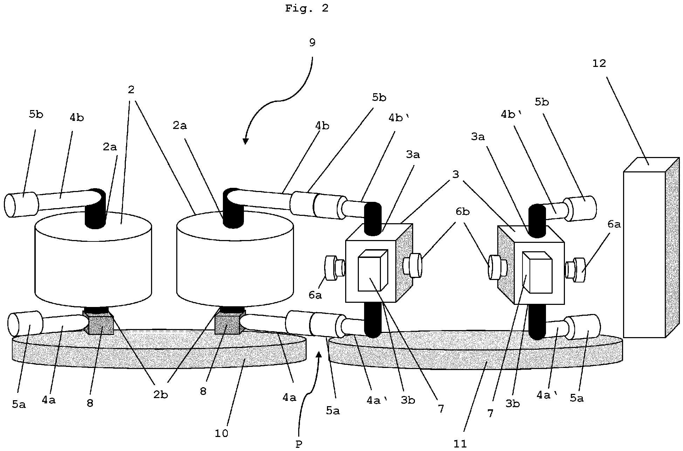

In said line (bypass), a treatment unit is provided where a cell material is continuously pumped through an arrangement of two electrodes and exposed to an electric field in an electrically conductive liquid. Such a device has been described in the above article by Buchmann L and Mathys A (2019), Perspective on Pulsed Electric Field Treatment in the Bio-based Industry, Front. Bioeng. Biotechnol. 7:265, doi: 10.3389/fbioe.2019.00265 (Fig.1), as well as by Galvan-D'Alessandro et al., Fermentation Assisted by Pulsed Electric Field and Ultrasound: A Review, Fermentation 2018, 4, 1; doi:10.3390 (Fig. 2).

In DE 10 2010 019 937 A1, a device for vitalization of yeast in a method for brewing beer is described. Two fermentation tanks are connected with each other via a bypass in which a unit for evoking electroporation is arranged.

The above devices known from the prior art are not optimal with respect to product throughput and treatment under hygienic conditions required for various medical and food applications. After the treatment of one certain material provided in the bioreactor, the devices known from the prior art have to be thoroughly cleaned and filled with new material before a new treatment process can be initiated.

It was the problem of the present invention to overcome the above drawbacks of the prior art.

The above problem is solved by the present invention as defined in the claims.

In detail, the present invention relates to a device, comprising a unit for taking up a fluid, a unit for emitting electric pulses, wherein said unit for taking up a fluid and said and optionally another unit for emitting electric pulses are connected with each other via lines so as to form a circuit, characterized in that there are provided connections for connecting the lines arranged at said and optionally another unit for taking up a fluid with the lines arranged at said unit for emitting electric pulses in a releasable, hygienic and leakage-free manner.

The present invention is based on the concept of providing a device for treating biological material by electric pulses in the form of separate units. This allows for a quick exchange of a single unit of said device, for example for cleaning purposes or for providing a new biological material to be treated without having first to empty and clean the unit for taking up a fluid (e.g. a bioreactor, fermenter and open pond).

According to the present invention, the unit for taking up a fluid may be any unit in which a fluid can be stored. With the device of the present invention, typically biological material in the form of a suspension is treated. Accordingly, the unit for taking up a fluid may be a unit for taking up and storing such a suspension. For example, said unit for taking up a fluid may be a cylindrical tank with at least one inlet and outlet suitable for inserting and discharging a suspension.

Preferably, said unit for taking up a fluid may be a bioreactor. A bioreactor is generally known in the art. A bioreactor is a device or system that supports a biologically active environment. Preferably, a bioreactor is a vessel in which a (bio)chemical process can be carried out which involves organisms or biochemically active substances derived from such organisms. This process can either be aerobic or anaerobic, for example fermentation. Conventional bioreactors range in size from litres to cubic metres, and are often made of stainless steel.

According to the present invention, said unit for taking up a fluid comprises lines arranged at said unit for taking up a fluid. According to the present invention, any line conventionally used for transporting a fluid may be used. Preferably, the lines are pipes, for example made from stainless steel, tubes or hoses.

According to a preferred embodiment of the present invention, said unit for taking up a fluid has an inlet connected to one of said lines which is formed in a top face of said unit, and an outlet connected to another of said lines which is formed in a bottom face of said unit. While it is principally possible to provide an inlet and/or outlet of said unit for taking up a fluid also in a side wall of said unit, providing said inlet and said outlet in a top face and bottom face of said unit is preferred for establishing a closed circuit in which the biological material to be treated can be circulated.

Other inlets and outlets may be provided in a similar manner to fill and empty the unit for taking up a fluid independently.

Preferably, at said inlet and/or said outlet units for regulating the flow may be provided. As an example, conventionally used valves or locks may be mentioned.

According to another embodiment of the present invention, two different units for taking up a fluid may be simultaneously connected to said one unit for emitting electric pulses. This allows the transfer of material to be treated from one unit for taking up a fluid to another unit for taking up a fluid, via said unit for emitting electric pulses.

The lines arranged at said unit(s) for taking up a fluid comprise at their remote ends from said unit(s) connections for connecting the lines arranged at said unit for taking up a fluid with lines arranged at a unit for emitting electric pulses in a releasable, hygienic and leakage-free manner.

These connections have to meet certain criteria to be suitable for the purposes of the present invention. For example, they have to be releasable in order to serve the purpose of the present invention that the unit(s) for taking up a fluid and the unit for emitting electric pulses can be disconnected from each other. Preferably, said connections should be releasable and re-connectable in a fast and easy manner. As an example, bayonet couplings are suitable couplings.

Also, said connection should be leakage-free, in order to avoid any waste or contamination of the biological material to be treated. This may be achieved, for example, by couplings having a plane front face closing an end of the line terminating in said connection, when the connection is in a disconnected state. Only when the connection between the parts of said connection is made, a fluidic connection is realized. With such connections, no leakage occurs during the connection and disconnection operation.

Furthermore, importantly said connection should connect the lines arranged at said unit(s) for taking up a fluid with lines arranged at a unit for emitting electric pulses in a hygienic manner. This is important in light of the susceptibility of the biological material to be treated to contaminations and the sanitary requirements imposed on the use of biological material in various applications. Suitable connections are leakage-free couplings which, as discussed above, prevent any leakage during the connection and disconnection operation, preferably by keeping the end of the line terminating in said connection closed, when the connection is in a disconnected state. In such way, material cannot exit from the line in the disconnected state, so that no contamination of the environment or unit(s) for taking up a fluid may occur. Also, no contamination may enter into the line where the biological material to be treated is transported, when the connection is in a disconnected state. Moreover, said connection is preferably made from smooth surfaces of an easily cleanable and sterilisable material, such as stainless steel.

The connection between the connection parts should be made connectible in such a way that a user wearing gloves can realize said connection in an easy and quick manner.

An example for a suitable connection according to the present invention is a so-called quick coupling. Quick couplings are known in the art and may be realized in the form of a bayonet coupling where the connection between the coupling parts may be realized by a simple pulling of one connection part into the other one and closing the connection by a simple turn of the connection part that has been pulled into the other one.
Such quick couplings are commercially available, for example the HCB couplings from Staubli, in particular HCB03, HCB05, HCB08, HCB12, HCB05, and HCB20.

Likewise, the lines arranged at said unit for emitting electric pulses comprise at their remote ends from said unit connections for connecting the lines arranged at said unit for emitting electric pulses with lines arranged at a unit for taking up a fluid in a releasable, hygienic and leakage-free manner. Said lines are preferably as discussed above, and end in the counterpart of said connection whose other part is arranged at the end of said lines arranged at a unit for taking up a fluid.

A unit for emitting electric pulses is known. Reference may be made, for example to applicant's co-pending applications EP20156005.9; EP 20156006.7; EP20156008.3; and EP 20156009.1.

According to a preferred embodiment of the present invention, said unit for emitting electric pulses comprises two or more electrodes or plates that are arranged in one electric circuit, and a treatment space arranged between said electrodes or plates so that the treatment space can be penetrated by the emitted electric pulses and an electric field resulting therefrom.

According to a preferred embodiment of the present invention, said unit for emitting electric pulses has an inlet connected to one of said lines arranged at said unit for emitting electric pulses which is formed in a top face of said unit, and an outlet connected to another of said lines arranged at said unit for emitting electric pulses which is formed in a bottom face of said unit. Alternative locations of the inlet and outlet are possible, as long as appropriate flow of the material to be treated through the treatment space is ensured.

Preferably, at said inlet and/or said outlet units for regulating the flow may be provided. As an example, conventionally used valves or locks may be mentioned.

According to a preferred embodiment of the present invention, the inlet comprises a nozzle or the inlet constitutes a nozzle. It is possible to use nozzles which can conventionally be used for introducing a medium into a container or defined space. Such nozzles are known.

With this embodiment, the speed at which the cell material or a suspension containing the cell material is introduced into the treatment unit can be adjusted in a targeted manner. The residence time of the cell material in the electric field applied to the treatment space of the treatment unit can thereby be adjusted.

The unit for emitting electric pulses into which the cell material to be treated is introduced through the inlet described above, is limited with respect to its shape, structure and dimensions only in that applied electric pulses and an electric field resulting therefrom can sufficiently penetrate the unit to achieve the desired treatment of the cell material in the unit. According to the invention, the unit for emitting electric pulses is preferably a container made of a metallic material or a plastic material (such as polyethylene terephthalate).

The unit for emitting electric pulses can have any geometric shape. Preferably, the unit for emitting electric pulses is a cuboid or cylindrical body.

A treatment space is provided within the unit for emitting electric pulses, through which treatment space the cell material passes and is thereby treated with electric pulses.

The treatment space can have any geometric shape, but preferably has a cuboid shape. According to a very preferred embodiment of the present invention, said treatment space is positioned in the centre of the unit for emitting electric pulses. Especially preferred the unit for emitting electric pulses is a solid body, and the treatment space is a cavity within said unit for emitting electric pulses, preferably in the centre of the unit for emitting electric pulses.

As described above, the unit for emitting electric pulses comprises an inlet and an outlet. The treatment space within the unit for emitting electric pulses is fluidly connected with the inlet and the outlet of the unit for emitting electric pulses.

The fluid connection of inlet, treatment space and outlet can be realized by providing conduits such as pipes that protrude from the inlet to the treatment space, and from the treatment space to the outlet. According to a preferred embodiment of the present invention where the unit for emitting electric pulses is a solid body, and the treatment space is a cavity within the unit for emitting electric pulses, the fluid connection of inlet, treatment space and outlet is realized by respective cavities in the unit for emitting electric pulses that protrude from the inlet to the treatment space, and from the treatment space to the outlet.

The fluid connection of inlet, treatment space and outlet can have a uniform shape, such as a cylindrical pipe or cavity with a uniform diameter. It is also possible that the shape of the fluid connection of inlet, treatment space and outlet varies over its length. For example, at least a part of the fluid connection may have a funnel-shaped form. For example, the fluid connection from the inlet to the treatment space may partially have a funnel-shaped form with an opening size decreasing from the inlet to the treatment space, so that the material may be advantageously guided into the treatment space. Likewise, the fluid connection from the treatment space to the outlet may partially have a funnel-shaped form with an opening size increasing from the treatment space to the outlet, so that the material may be advantageously guided to the outlet.

According to a preferred embodiment of the present invention, at least a portion of an inner side face of the treatment space of said unit for emitting electric pulses is non-planar, preferably curved. It has been found that more homogeneous treatment is possible in a treatment space that has an inner side face with at least a portion thereof being non-planar. For example, said portion of said inner side face may be curved, triangular, baffled or may have a saw-tooth shape. A curved portion of said inner side face is preferred. The flow of a fluid protruding through said treatment space is thereby significantly homogenized in a zone where the electric field is applied, and as a result treatment of a material flowing through said treatment space, by means of electric pulses, is significantly more homogenous than in a treatment space having plain (i.e. not curved) side faces.

It is preferred that said at least a portion of an inner side face of the treatment space has a regular surface shape. Especially preferred, said inner side face of the treatment space has a sinusoidal shape, at least over a portion thereof.

According to the present invention, a preferred design of the treatment space is such that two side surfaces of the treatment space which lie opposite to each other are curved, and preferably have a sinusoidal shape, over at least 50%, preferably at least 75% and most preferably 100% of their respective heights. Especially preferable is a sinusoidal shape with an amplitude in the range from 5 to 20 %, preferably 10 to 15%, of the width of the treatment space, and a wavelength in the range from 50 to 80%, preferably 60 to 70% of the height of the treatment space. So, for example, for a treatment space having a dimension of 24x5x5 mm, a preferred curvature would have a wavelength of 13 mm (i.e. about 66.67% of the height of the treatment space) and an amplitude of 0.67 mm (i.e. about 13.4% of the width of the treatment space).

According to another preferred embodiment of the present invention, there are provided two side surfaces of the treatment space which lie opposite to each other and have a sinusoidal shape such that there is a phase shift of 180°C between the two side surfaces.

According to an especially preferred embodiment of the present invention, at least one, preferably two side faces of a cuboid treatment space have a curved, preferably sinusoidal shape over the entire height. Especially preferred, these two side faces of the cuboid treatment space lie opposite to each other.

With the above described design of the treatment space, the flow of a material through the treatment space become more homogenous due to flow modifications outside the region of the treatment zone where electric pulses are applied. Accordingly, there is less difference between the flow (residence time) of the material in the middle of the treatment space and the flow of the material in vicinity of the side surfaces of the treatment space. Accordingly, electric pulses emitted through the treatment space, and an electric field resulting therefrom, contact a material with more homogeneous characteristics and thus result in a more homogeneous treatment of said material.

According to a preferred embodiment of the present invention, said device further comprises a conveying unit such as a pump for conveying the fluid through the circuit formed by said unit(s) for taking up a fluid, said unit for emitting electric pulses, and said lines which are arranged at the respective units and connected with each other by the connections described herein.

The electric pulses to penetrate the treatment space are generated and emitted by electrodes, preferably two electrodes, which are positioned at the unit for emitting electric pulses such that the electric pulses emitted by said electrodes, and thus the electric field resulting therefrom, may sufficiently penetrate the treatment space and thus enable adequate treatment of the material in the treatment space. Alternatively, two plates e.g. of a capacitor may be used for this purpose.

According to a preferred embodiment of the present invention, in the region of the treatment space there are provided means for positioning electrodes, or plates e.g. of a capacitor, for providing said electric pulses to the treatment space. Those means may be any known component enabling adequate and secure fixation of the electrodes or plates at the treatment unit. For example, fixation tools such as clamping tools may be provided at side faces, preferably opposite side faces, of the treatment unit, so that the electrodes or plates may be securely fixed at the side surfaces of the unit for emitting electric pulses.

According to a very preferred embodiment of the present invention, the means for positioning electrodes are openings in the side faces preferably opposite side faces, of the unit for emitting electric pulses into which electrodes may be inserted. These openings have a size allowing insertion of conventional electrodes and securing the inserted electrodes at place. These openings preferably are located in the vicinity of the treatment space in the unit for emitting electric pulses.

For example, the means may be blind bores in side faces of the unit for emitting electric pulses, which is preferably a solid body. In a preferred embodiment, blind bores are provided in two side faces of the unit for emitting electric pulses opposite to each other. Preferably, these blind bores are located in the vicinity of the treatment space in the unit for emitting electric pulses. It is very preferred that the blind bores have such a depth that their proximal face with respect to the treatment space lies close to a side face of the treatment space. Especially preferred, a side face of the treatment space forms the end face of a blind bore.

According to a preferred embodiment, the blind bores may be tapered from the outside to the inside end, i.e. the opening size of the blind bore decreases from the outside to the inside. This allows secure fixation of an electrode inserted into said blind bore.

In order to not disturb the penetration of electric pulses generated and emitted by said electrodes, preferably the inner face of the blind bores comprises a slit, which preferably is arranged in the centre of the inner face of the blind bore. At the position of the slit, the separating wall between the blind bore and the treatment space is thus thinner. The exact extension of the slit is to be chosen by a skilled person that the remaining side face of the treatment space withstands the flow conditions during the treatment period.

According to a further preferred embodiment, the blind bores (or more generally the means for positioning the electrodes) project towards plain side faces of the treatment space. In other words, according to this embodiment side faces of the treatment space that have a curved inner portion do not lie in vicinity of the electrodes generating and emitting electric pulses.

The device of the present invention also comprises a unit for generating electric pulses. Units for generating electric pulses are well known. According to the present invention, devices which can generate electrical impulses as described below are preferred. Such devices are known. By way of example, cable pulse generators, semiconductor-based pulse generators, or relaxation oscillators can be mentioned.

The generated electric pulses are emitted into the treatment space. This is preferably done by two or more electrodes or plates e.g. of a capacitor arranged parallel to each other, which are electorally connected with said unit for generating electric pulses, and which are arranged opposite each another having a distance suitable for the generation of electric pulses. Such arrangements are well known and need not be explained in detail here.

According to a preferred embodiment of the present invention, two electrodes or plates e.g. of a capacitor are arranged perpendicularly to the direction of movement of the material through the treatment space.

According to the present invention, the inner portion of the treatment space, i.e. not the portions thereof which are in direct vicinity to the non-planar portion(s) of said inner side face(s), are subjected to electric pulses and to an electric field generated therefrom.

The electric field to be applied to the treatment space must be characterized such that it provides for the desired effect, e.g. that it stimulates the growth of the treated cells. Corresponding electric fields are known from the prior art, for example from EP-2 308 969 B1. According to the present invention, an electric field generated from such electric pulses can be used, such that a voltage increase takes place between the two electrodes or plates of a capacitor of the device of 10% to 90% of a target voltage of the electric pulses within a period of 0.1 to 1000 ns, the electric pulses have a pulse duration of 5 ns to 50000 ns, and the electric pulses, upon reaching the target voltage, have an electric field strength of 0.5 kV/cm to 100 kV/cm.

According to the present invention, said device is suitable for medical, environmental, food applications, and bio-based industries (including yeast, lactobacilli, algae, and cell tissue production systems, in particular including targeted inactivation, the extraction of bioactive compounds, and the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe.2019.00265.

According to the present invention, electric pulses are applied to a cell material located in the treatment space of a treatment unit while it passes through the treatment space. As a result, the cell material is subjected to the electric pulses and treated.

According to the present invention, basically any material composed of at least one cell, that is, both eukaryotic and prokaryotic cells, can be used as the cell material to be treated. The cell material can be unicellular or multicellular organisms. Examples would be bacteria, yeasts, microalgae, plant cells, and fungal cells or their spores, mycelia, seeds or seedlings and somatic animal cells. Furthermore, multicellular tissues such as meristems in plants and epithelial or connective tissue in humans or animals can be treated.

The cell material is usually (but not necessarily) isolated, purified and/or sterilized in a known manner before being treated according to the invention. Preferably, the cell material can already be propagated in a known manner in suitable and known culture media to a desired degree before the treatment according to the invention.

The cell material is preferably suspended in an electrically conductive liquid prior to the treatment according to the invention. Electrically conductive liquids are well known. According to the invention, it is necessary to use electrically conductive liquids which have no adverse effects on cell viability, that is, in particular, are non-toxic. According to the invention, water is preferably used as the electrically conductive liquid, wherein the water can be adjusted to a desired pH value by means of suitable and known additives. According to the invention, a pH value in the range of 6.0 to 14.0, preferably 7 to 12 is preferred.

According to the present invention, the suspensions described above can be prepared in a conventional manner and stored until treatment. However, the suspensions can also be provided immediately before the treatment according to the invention.

The cell material or a suspension containing the cell material is passed through the inlet of a treatment unit into a treatment space which is located inside the unit for emitting electric pulses.

The present invention is furthermore related to an apparatus for treating cells for targeted inactivation, the extraction of bioactive compounds, and the stimulation of cell growth and/or cellular compounds, comprising a device as described herein above, and additional units for taking up a fluid, preferably 2 to 10 additional units for taking up a fluid, that are arranged such that they can be sequentially connected in a releasable, hygienic and leakage-free manner with said unit for emitting electric pulses, and/or additional units, preferably one additional unit, for emitting electric pulses.

As explained above, the concept of the present invention is to provide a device for treating biological material in a form of units that can be releasably connected and also disconnected. This allows for a quick exchange of a single unit of said device, for example for cleaning purposes or for providing a new biological material to be treated without having first to empty and clean the unit for taking up a fluid (e.g. a bioreactor). Accordingly, the apparatus of the present invention comprises, beside a device as described herein above, additional units for taking up a fluid and/or additional units for emitting electric pulses.

According to a preferred embodiment, 2 to 10 additional units for taking up a fluid are provided that are arranged such that they can be sequentially connected in a releasable, hygienic and leakage-free manner with said unit for emitting electric pulses.

In other words, a unit for taking up a fluid that is connected to a unit for emitting electric pulses can be disconnected from said unit for emitting electric pulses, by releasing the connections described above, and is then moved away from the position where it was in connection with said unit for emitting electric pulses. Said position is called connecting position. Then, another unit for taking up a fluid can be moved into the connecting position and connected with said unit for emitting electric pulses, by closing the connections described above.

In this way, the efficiency is significantly increased. If a large amount of one biomaterial, or different biomaterials are to be treated, fresh biomaterial can be brought to the treatment zone in the unit for emitting electric pulses by connecting a new unit for taking up a fluid, for example a bioreactor, to said unit for emitting electric pulses. There is no need for a longer interruption of the process, for example due to cleaning a single available unit for taking up a fluid and refilling the same.

According to another embodiment of the present invention, two different units for taking up a fluid may be simultaneously connected to said one unit for emitting electric pulses. This allows the transfer of material to be treated from one unit for taking up a fluid to another unit for taking up a fluid, via said unit for emitting electric pulses.

According to a preferred embodiment of the present invention, said units for taking up a fluid are movably arranged, preferably on a wheeled device, so that they can sequentially be moved into a connecting position where they can be connected in a releasable, hygienic and leakage-free manner with said unit or one of said units for emitting electric pulses.

Any movable arrangement which allows bringing the units for taking up a fluid into the connecting position is suitable for the purpose of the present invention. For example, a wheeled device or a conveyor belt may be mentioned.

According to the present invention, a wheeled device is a circular device which can be rotated. Preferably, said rotational movement is evoked by a motor that can preferably be arranged underneath said wheeled device. On the top surface of the wheeled device, said units for taking up a fluid may be arranged, preferably releasably arranged. Such a wheeled device is generally known.

According to a preferred embodiment of the present invention, said units for emitting electric pulses are movably arranged, preferably on a wheeled device, so that they can sequentially be moved into a position where they can be connected in a releasable, hygienic and leakage-free manner with one of said units for taking up a fluid.

With this embodiment, also the efficiency is significantly increased. In the case of necessity of cleaning said unit for emitting electric pulses, the soiled unit for emitting electric pulses can be disconnected from said unit for taking up a fluid and brought into a remote position, where it can be cleaned. In the meantime, an additional unit for emitting electric pulses can be brought into the connecting position and connected with a unit for taking up a fluid, for example a bioreactor. There is no need for a longer interruption of the process.

Any movable arrangement which allows bringing the units for emitting electric pulses into the connecting position is suitable for the purpose of the present invention. For example, a wheeled device or a conveyor belt may be mentioned.

According to the present invention, a wheeled device is a circular device which can be rotated. Preferably, said rotational movement is evoked by a motor that can preferably be arranged underneath said wheeled device. On the top surface of the wheeled device, said units for emitting electric pulses may be arranged, preferably releasably arranged. Such a wheeled device is generally known.

According to another preferred embodiment of the present invention, said apparatus further comprises a cleaning unit for cleaning, preferably sterilization, of said unit for emitting electric pulses and/or of said unit for taking up a fluid, and/or of said lines and/or said connections.

Such a cleaning unit is generally known. Preferably, it is located at a position remote from the connecting position described above.

The present invention is furthermore related to a unit for emitting electric pulses, which is connectable to a unit for taking up a fluid via lines so as to form a circuit, **characterized in that** there are provided connections for connecting the lines arranged at said unit for taking up a fluid with the lines arranged at said unit for emitting electric pulses in a releasable, hygienic and leakage-free manner.
The present invention is furthermore related to a method for treating cells for targeted inactivation, the extraction of bioactive compounds, and the stimulation of cell growth and/or cellular compounds, performed in a device or in an apparatus as described above, comprising the steps
a) applying electric pulses to a treatment space in a unit for emitting electric pulses,
b) introducing cell material, preferably as a suspension in an electrically conductive liquid, from a unit for taking up a fluid into the treatment space,
c) passing of the cell material through the treatment space and the electric pulses penetrating the treatment space and conveying said cell material back into said or optionally another unit for taking up a fluid.

The method can be performed as already described above.
As stated above, it is preferred that the cell material is provided as a suspension in an electrically conductive liquid.

As stated above, it is further preferred thatan electric field is applied with such electric field strength pulses, so that a voltage increase takes place between the two electrodes (6a, 6b) or plates of 10% to 90% of a target voltage of the electric field strength pulses within a period of 0.1 to 1000 ns, the electric field strength pulses have a pulse duration of 5 ns to 50000 ns, and the electric field strength pulses, upon reaching the target voltage, have an electric field strength of 0.5 kV/cm to 100 kV/cm.

The treatment process of the present invention may be performed continuously or batch-wise. In the latter case, the unit for emitting electric pulses may be emptied before another treatment cycle is started. Optionally, a sterilization/sanitization step may be performed prior to the start of another treatment cycle. Known sterilization/ sanitization processes such as steam-based processes can be used in accordance with the present invention.

According to a preferred embodiment of the present invention, after steps a) to c) have been performed, said unit (s) for taking up a fluid and said unit for emitting electric pulses are disconnected from each other by releasing the connections, another (one) of said units for taking up a fluid or of said units for emitting electric pulses is/are moved into a connecting position, a releasable, hygienic and leakage-free connection of the unit(s) for taking up a fluid and the unit for emitting electric pulses that are in the connecting position is realized via the connections, and steps a) to c) are repeated.

According to a preferred embodiment of the present invention, after steps a) to c) have been performed, said unit for emitting electric pulses and/or of said lines and/or said connections are cleaned, preferably sterilized.

The present invention further relates to the use of the device or apparatus according to the present invention described herein for medical, environmental, food applications, and bio-based industries (including yeast, bacteria, microalgae, as well as plant or animal cells and cell tissue production systems, in particular targeting inactivation, the extraction of bioactive compounds, and/or the stimulation of cell growth and/or cellular compounds. These applications are described in Buchmann L and Mathys A (2019), Perspective on Pulsed Electric Field Treatment in the Bio-based Industry, Front. Bioeng. Biotechnol. 7:265, doi: 10.3389/fbioe. 2019.00265.

The present invention is explained below by way of non-limiting examples and figures.
- Fig. 1: shows a schematic representation of an embodiment of the device of the present invention
- Fig. 2: shows a schematic representation of an embodiment of the apparatus of the present invention
- Fig. 3: a schematic representation of the unit for emitting electric pulses of the embodiment of the device of the present invention according to Fig. 1
- Fig. 4a: a side view of the unit for emitting electric pulses of the embodiment of the device of the present invention according to Fig. 1
- Fig. 4b: another schematic representation of the unit for emitting electric pulses of the embodiment of the device of the present invention according to Fig. 1.
In the figures, same references numbers designate the same components.

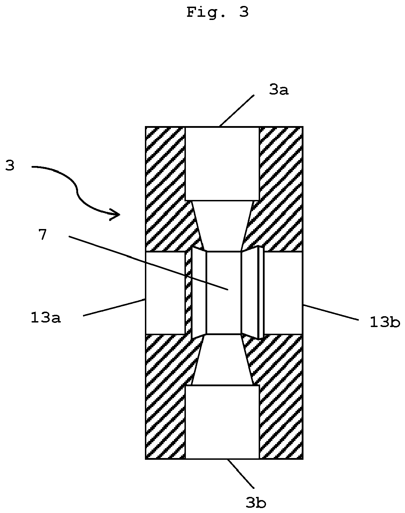

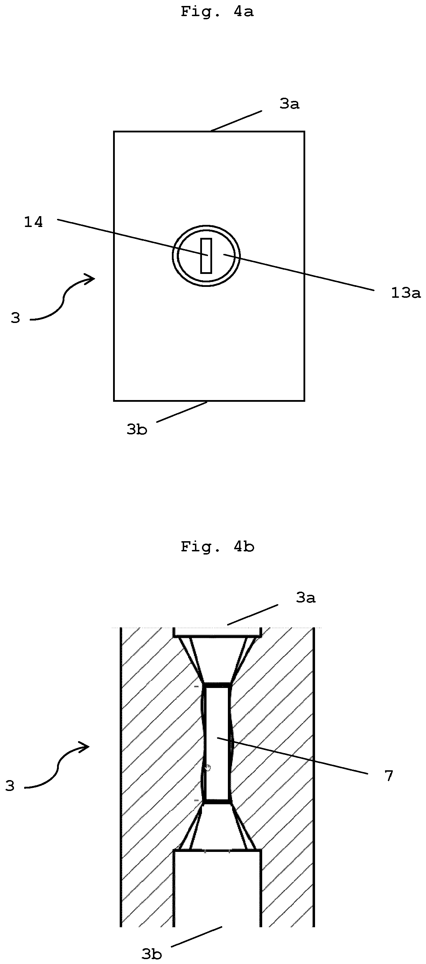

In Fig. 1, a schematic representation of an embodiment of the device 1 of the present invention is shown. Said embodiment comprises a unit 2 for taking up a fluid, here in the form of a cylinder. Said unit 2 for taking up a fluid may be a bioreactor. The device 1 according to the embodiment of Fig. 1 furthermore comprises a unit 3 for emitting electric pulses, which will be discussed below in more detail with respect to Figs. 3, 4a and 4b. Said unit 3 for emitting electric pulses comprises electrodes 6a, 6b, and a treatment zone 7 within said unit 3 for emitting electric pulses. The electrodes 6a, 6b are connected to a unit for generating electric pulses (not shown) via electric conduits such as cables.

In the embodiment of Fig. 1, lines 4a, 4b are arranged at said unit 2 for taking up a fluid. Said unit 2 for taking up a fluid has an inlet 2a that is connected to line 4b and that is formed in a top face of said unit 2, and an outlet 2b connected to another line 4a and that is formed in a bottom face of said unit 2.

In the embodiment of Fig. 1, lines 4a', 4b' are arranged at said unit 3 emitting electric pulses. Said unit 3 for emitting electric pulses has an inlet 3a that is connected to line 4b' and that is formed in a top face of said unit 3, and an outlet 3b connected to another line 4a' and that is formed in a bottom face of said unit 3.

Connections 5a, 5b are provided for connecting the lines 4a, 4b arranged at said unit 2 for taking up a fluid with the lines 4a', 4b' arranged at said unit 3 for emitting electric pulses in a releasable, hygienic and leakage-free manner. In the embodiment of Fig. 1, said connections 5a, 5b are preferably quick couplings as described above.

In the embodiment of Fig. 1, there is also provided a conveying unit 8 in the form of a pump that conveys the biomaterial to be treated through the circuit formed by the unit 2 for taking up a fluid, lines 4a, 4a', 4b, 4b', and the unit 3 for emitting electric pulses.

In Fig. 2, a schematic representation of an embodiment of the apparatus 9 of the present invention is shown. Said embodiment comprises two units 2 for taking up a fluid and two units 3 for emitting electric pulses, with the additional components arranged thereto (lines, inlet, outlet, pump, and connection parts). These two units 2 for taking up a fluid are arranged on a rotatable wheeled device 10 that can be moved by a motor. The two units 2 for taking up a fluid are preferably releasably arranged on said wheeled device 10, for example by suitable fixing means such as screw connections(not shown). These two units 3 for emitting electric pulses are arranged on a rotatable wheeled device 11 that can be moved by a motor. The two units 3 for emitting electric pulses are preferably releasably arranged on said wheeled device 11, for example by suitable fixing means such as screw connections(not shown).

In Fig. 2, one unit 2 for taking up a fluid and one unit 3 for emitting electric pulses are positioned in the connecting position P, and connected by closing the connections 5a, 5b. They thus form the device 1 shown in Fig. 1. The other unit 2 for taking up a fluid and the other unit 3 for emitting electric pulses are positioned outside the connecting position P and are disconnected.

A cleaning unit 12 is provided that can be moved so as to clean a component of the apparatus 9, as described above.

Fig. 3 is a schematic representation of the unit 3 for emitting electric pulses of the embodiment of the device 1 of the present invention according to Fig. 1.

In the embodiment of Fig. 3, the unit 3 for emitting electric pulses is a solid body with a treatment space 7 in the form of a cavity in its center. The inlet 3a in the top face of the unit 3 for emitting electric pulses is fluidly connected, by means of a conduit, with the treatment space 7. In this embodiment, the fluid connection is funnel-shaped over its lower part discharging into the treatment space 7. The outlet 3b in the bottom face of the unit 3 for emitting electric pulses is also fluidly connected, by means of a conduit, with the treatment space 7. In this embodiment, the fluid connection is funnel-shaped over it upper part extending from the treatment space 7.

In two side faces of the unit 3 for emitting electric pulses, there are provided openings 13a, 13b in the form of blind bores for insertion of electrodes. These openings are tapered in a region adjacent to the treatment space 7, i.e. they become smaller as they approach the treatment space 7. The side faces of the treatment space 7 adjacent to the openings 13a, 13b are plain, i.e. they are not curved.

Fig. 4a is a side view of the unit 3 for emitting electric pulses of the embodiment of the device (1) of the present invention according to Fig. 1.

In Fig. 4a, a side face of the unit 3 for emitting electric pulses is shown in which there is provided an opening 13a in the form of a blind bore. Said blind bore is tapered in a region adjacent to the treatment space (7, not shown here), i.e. it becomes smaller with increasing depth. In the inner face of the opening 13a, preferably in the center of the inner face, there is provided a slit 14.

Fig. 4b is another schematic representation of the unit 3 for emitting electric pulses of the embodiment of the device (1) of the present invention according to Fig. 1.

As compared to Fig. 3, the unit 3 for emitting electric pulses is turned by 90°. Thus, in Fig. 4b the side faces of the treatment space 7 are shown that are not adjacent to the openings 13a, 13b. These side faces are curved. Here, they have a sinusoidal shape over their entire height.

## Claims

1. A device (1) for treating cells, comprising
a unit (2) for taking up a fluid,
a unit (3) for emitting electric pulses, wherein said unit (2) for taking up a fluid and said and optionally another unit (3) for emitting electric pulses are connected with each other via lines (4a, 4a', 4b, 4b') so as to form a circuit,
**characterized in that** there are provided connections (5a, 5b) for connecting the lines (4a, 4b) arranged at said and optionally said other unit (2) for taking up a fluid with the lines (4a', 4b') arranged at said unit (3) for emitting electric pulses in a releasable, hygienic and leakage-free manner.

2. The device according to claim 1, **characterized in that** said unit (3) for emitting electric pulses comprises two or more electrodes or plates (6a, 6b) that are arranged in one electric circuit, and a treatment space (7) arranged between said electrodes or plates (6a, 6b) so that the treatment space (7) can be penetrated by the emitted electric pulses and an electric field resulting therefrom.

3. The device according to any of the preceding claims, **characterized in that** said unit (3) for emitting electric pulses has an inlet (3a) connected to one of said lines (4b') which is formed in a top face of said unit (3), and an outlet (3b) connected to another of said lines (4a') which is formed in a bottom face of said unit (3).

4. The device according to any of the preceding claims, **characterized in that** said unit(s) (2) for taking up a fluid has/have an inlet (2a) connected to one of said lines (4b) which is formed in a top face of said unit (2), and an outlet (2b) connected to another of said lines (4a) which is formed in a bottom face of said unit (2).

5. The device according to any of the preceding claims, **characterized in that** said device further comprises a conveying unit (8) such as a pump for conveying the fluid through the circuit formed by said unit(s) (2) for taking up a fluid, said unit (3) for emitting electric pulses, and said lines (4a, 4a', 4b, 4b').

6. The device according to any of the preceding claims, **characterized in that** said connections (5a, 5b) for connecting the lines (4a, 4b) arranged at said unit(s) (2) for taking up a fluid with the lines (4a', 4b') arranged at said unit (3) for emitting electric pulses in a releasable and leakage-free manner are quick couplings.

7. The device according to any of the preceding claims, **characterized in that** said unit (3) for emitting electric pulses can emit electric field strength pulses, so that a voltage increase take place between the two or more electrodes (6a, 6b) of 10% to 90% of a target voltage of the electric field strength pulses within a period of 0.1 to 1000 ns, the electric field strength pulses have a pulse duration of 5 ns to 50000 ns, and the electric field strength pulses, upon reaching the target voltage, have an electric field strength of 0.5 kV/cm to 100 kV/cm.

8. An apparatus (9) for treating cells for targeted inactivation, the extraction of bioactive compounds, and the stimulation of cell growth and/or cellular compounds, comprising a device (1) according to any of claims 1 to 7, and additional units (2) for taking up a fluid, preferably 2 to 10 additional units (2) for taking up a fluid, that are arranged such that they can be sequentially connected in a releasable, hygienic and leakage-free manner with said unit (3) for emitting electric pulses, and/or additional units (3), preferably one additional unit (3), for emitting electric pulses.

9. The apparatus according to claim 8, **characterized in that** said units (2) for taking up a fluid are movably arranged, preferably on a wheeled device (10), so that they can sequentially be moved into a connecting position (P) where they can be connected in a releasable, hygienic and leakage-free manner with said unit (3) or one of said units (3) for emitting electric pulses.

10. The apparatus according to claim 8, **characterized in that** said units (3) for emitting electric pulses are movably arranged, preferably on a wheeled device (11), so that they can sequentially be moved into a connecting position (P) where they can be connected in a releasable, hygienic and leakage-free manner with one of said units (2) for taking up a fluid.

11. The apparatus according to any of claims 8 to 20, **characterized in that** said apparatus further comprises a cleaning unit (12) for cleaning, preferably sterilization, of said unit (3) for emitting electric pulses, and/or of said unit (2) for taking up a fluid and/or of said lines (4a, 4a', 4b, 4b') and/or said connections (5a, 5b).

12. A method for treating cells for targeted inactivation, the extraction of bioactive compounds, and the stimulation of cell growth and/or cellular compounds, performed in a device (1) according to any one of claims 1 to 7 or in an apparatus (9) according to any of claims 8 to 11, comprising the steps
a) applying electric pulses to a treatment space (7) in a unit (3) for emitting electric pulses,
b) introducing cell material, preferably as a suspension in an electrically conductive liquid, from a unit (2) for taking up a fluid into the treatment space (7),
c) passing of the cell material through the treatment space (7) and the electric pulses penetrating the treatment space (7) and conveying said cell material back into said or optionally another unit (2) for taking up a fluid.

13. The method according to claim 12, **characterized in that** after steps a) to c) have been performed, said unit(s) (2) for taking up a fluid and said unit (3) for emitting electric pulses are disconnected from each other by releasing the connections (5a, 5b), at least another one of said units (2) for taking up a fluid or of said units (3) for emitting electric pulses is moved into a connecting position (P), a releasable, hygienic and leakage-free connection of the unit (2) for taking up a fluid and the unit (3) for emitting electric pulses that are in the connecting position (P) is realized via the connections (5a, 5b), and steps a) to c) are repeated.

14. The method according to claim 12 or 13, **characterized in that** after steps a) to c) have been performed, said unit (3) for emitting electric pulses and/or of said lines (4a, 4a', 4b, 4b') and/or said connections (5a, 5b) are cleaned, preferably sterilized.

15. The method according to any of claims 12 to 14, **characterized in that** an electric field is applied with such electric field strength pulses, so that a voltage increase takes place between the two electrodes (6a, 6b) or plates of 10% to 90% of a target voltage of the electric field strength pulses within a period of 0.1 to 1000 ns, the electric field strength pulses have a pulse duration of 5 ns to 50000 ns, and the electric field strength pulses, upon reaching the target voltage, have an electric field strength of 0.5 kV/cm to 100 kV/cm.
